# EUROPÄISCHE PATENTSCHRIFT

(11) **EP 2 029 121 B1**
(45) Veröffentlichungstag und Bekanntmachung des Hinweises auf die Patenterteilung: **03.03.2010**
(21) Anmeldenummer: 07725656.8
(22) Anmeldetag: 30.05.2007
(51) Int. Cl.: A61K 9/70

(54) **WIRKSTOFFPARTIKELHALTIGES TRANSDERMALES THERAPEUTISCHES SYSTEM MIT ERHÖHTEM WIRKSTOFFFLUSS**
TRANSDERMAL THERAPEUTIC SYSTEM COMPRISING ACTIVE INGREDIENT PARTICLES AND HAVING INCREASED ACTIVE INGREDIENT FLUX
SYSTÈME THÉRAPEUTIQUE PERCUTANÉ CONTENANT DES PARTICULES DE PRINCIPE ACTIF AVEC UN FLUX DE PRINCIPE ACTIF ACCRU

(30) Priorität: 08.06.2006 DE 102006026578
(43) Veröffentlichungstag der Anmeldung: 04.03.2009
(73) Patentinhaber: LTS LOHMANN Therapie-Systeme AG, 56626 Andernach (DE)
(72) Erfinder: HORSTMANN, Michael, 56564 Neuwied (DE); SAMETI, Mohammad, 53177 Bonn (DE); JUNG, Tobias, 79588 Efringen-Kirchen (DE)
(74) Vertreter: Schweitzer, Klaus
(86) Internationale Anmeldenummer: PCT/EP2007/004765
(87) Internationale Veröffentlichungsnummer: WO 2007/140909

(56) Entgegenhaltungen:
- EP-A- 0 391 172
- EP-A- 0 481 443
- WO-A-00/47191

## Beschreibung

Die Erfindung betrifft transdermale therapeutische Systeme mit eingeschlossenem partikulärem Wirkstoff (Einschlusskörpern), welcher durch Feuchtigkeit, beispielsweise Hautfeuchtigkeit, aktivierbar ist, sowie Verfahren zur Herstellung solcher Systeme.

Transdermale Therapeutische Systeme (TTS) sind seit einer Reihe von Jahren in der Fachwelt bekannt und im Markt eingeführt. Transdermale therapeutische Systeme sind auf der Haut aufzubringende, selbstklebende galenische Zubereitungen mit festgelegter Applikationsfläche, die einen Arzneistoff nach Zeit und Menge kontrolliert an den menschlichen oder tierischen Körper abgeben.

Der therapeutische Fortschritt dieser Systeme gegenüber traditionellen Applikationsformen besteht darin, dass der Wirkstoff dem Körper nicht stoßweise zugeführt wird, wie beispielsweise bei Einnahme von Tabletten, sondern kontinuierlich.

Dadurch wird einerseits die Wirkungsdauer eines Arzneistoffes verlängert, zum anderen werden Nebenwirkungen durch Vermeidung unnötiger Blutspiegelspitzen weitgehend verhindert.

Für solche Systeme werden üblicherweise geschichtete, flache Formen unter Verwendung verschiedener Polymere eingesetzt, unter denen Polyethylenterephthalat, Polyisobutylen, Polysiloxan beispielhaft erwähnt sind.

Zum Zwecke der verbesserten Klebkraft auf feuchten Oberflächen können neben zahlreicher, dem Fachmann bekannter Stoffen (Harzen, Ölen, Füllstoffen, Stabilisatoren) auch wasserlöslich / quellbare Zusätze eingebracht werden (EP 0307187). Auch nanopartikuläre Hilfsstoffe sind als Hilfsstoffe für die transdermale Abgabe versuchsweise eingesetzt worden (J. Microencaps. (1991), p. 369-374), wenn auch mit beschränktem Erfolg. Neben den üblicheren polymeren Hilfsstoffen sind darüber hinaus nanostrukturierte Lipidträger für Wirkstoffe versucht worden, z.B. mit Indomethacin (J. Pharm. Sei. (2005), p. 1149-1159).

In der Frühzeit der transdermalen Systeme herrschte unter Experten die Meinung vor, die Hauptschwierigkeit der Abgabe über die Haut bestehe in der Kontrollbedürftigkeit der Abgabegeschwindigkeit. Aus diesem Grunde sind den Wirkstoff und u. a. auch den optionalen Resorptionsverstärker kontrollierende Membranen in solche Systeme eingebracht worden (hier z.B. US 4,460,372). Auch wurde versucht, die Abgabekontrolle über unterschiedlich dimensionierte Partikelgrößen bis hin zu Mikro- und Nanopartikeln auf diese Weise zu steuern (US 4,687,481).

Da die menschliche Haut jedoch nicht für alle in Betracht kommenden Arzneistoffe eine ausreichende Durchlässigkeit aufweist ist in transdermalen therapeutischen Systemen herkömmlicher Art lediglich eine geringe Anzahl von Wirkstoffen einsetzbar. Es sind daher zahlreiche Versuche mit dem Ziel unternommen worden, die natürliche Durchlässigkeit der Haut zu steigern.

Eine solche Möglichkeit ist die Verwendung von so genannten Penetrationsverstärkern oder Resorptionsförderern. Man versteht hierunter Stoffe, die durch chemisch-physikalische Wechselwirkung mit der Mikrostruktur der Haut eine deutliche Steigerung des Wirkstoffflusses erzielen. Viele dieser Stoffe wirken jedoch auf der Haut toxisch oder verursachen Irritationen. Auch setzt die Wirkung dieser Resorptionsförderer nicht immer schnell genug ein, so dass der Effekt schwer zu steuern ist.

Eine andere Möglichkeit ist die Anwendung physikalischer Prinzipien, wie beispielsweise der lonophorese, der ultraschallgestützten Permeationsverstärkung (Sonophorese) oder auch der Anwendung von Mikronadeln (z.B. US 3,964,482). Diese Verfahren erfordern jedoch vergleichsweise aufwendige Zusatzeinrichtungen im transdermalen therapeutischen System, welche in aller Regel diese Form der Therapie unwirtschaftlich machen.

Ein grundsätzlich anderer Weg, die Hautdurchlässigkeit zu erhöhen, liegt in der Erhöhung der thermodynamischen Aktivität des Wirkstoffes. Diesbezügliche Versuche zielen auf eine Erhöhung der von außen wirkenden Konzentration des Wirkstoffs ab, um die Permeation zu steigern. Diese Bemühungen fanden ihre Grenze darin, dass im allgemeinen die Konzentration eines Wirkstoffs nicht über die Sättigungslöslichkeit hinaus gesteigert werden kann. Zum anderen führt es auch nicht weiter, im transdermalen therapeutischen System galenische Grundlagen mit höherer Löslichkeit für den Wirkstoff zu benutzen, da hier die Verknüpfung zwischen Verteilungskoeffizient und Löslichkeit gemäß dem Nernstschen Verteilungsgesetz zum Tragen kommt und limitierend wirkt.

Vorübergehend können so genannte übersättigte Zustände entstehen, bei denen die gelöste Wirkstoffkonzentration über der Sättigungskonzentration liegt, z.B. beim Abkühlen einer gesättigten Lösung. Solche System sind z.B. in US 5,174,995 beschrieben, bei denen gesättigte Lösungen von Wirkstoffen auf die Haut gebracht werden und, durch den Einfluss der löslichkeitsvermindernden Wirkung der Hautfeuchte, zu Übersättigung und damit gesteigertem Wirkstofftransport führen. Es liegt auf der Hand, dass das Ausnutzen solcher Zustände in Flüssigkeiten durch Ausfällung der Wirksubstanz und demzufolge verminderte Konzentrationen und Abgaberaten schnell zum Erliegen kommt. Länger als in Lösungen flüssiger Medien können übersättigte Zustände in transdermal üblichen klebenden Polymeren erzeugt und aufrechterhalten werden (J. Pharm.Sei. (2004), p. 2039-2048). Hier wurde eine Konzentration von bis zu dem Vierfachen der Sättigungslöslichkeit für Minuten bis Tage erfolgreich aufrecht erhalten. Aber auch diese Stabilität reicht bei Weitem nicht für vermarktbare transdermale Systeme aus. Bei bestimmten Wirkstoffen, deren Schmelzpunkt dicht oberhalb der Raumtemperatur liegt, wie z.B. Scopolamin lassen sich solche Übersättigungen allerdings unter Umständen durch herstellungstechnische Maßnahmen ausreichend lange stabilisieren (US 6,238,700).

Für partikulär vorliegende Wirkstoffe, deren Schmelzpunkt deutlich über der Raumtemperatur (somit also über ca. 50 °C) liegt, lässt sich mit dieser vorgeschlagenen Lösung kein stabiles System erzielen. Durch die Kombination einer den Hautfeuchtigkeitszutritt limitierenden Schicht und einer Matrix mit wasserunlöslichem Basismaterial und darin enthaltenen Einschlüssen, welche wiederum den Wirkstoff enthalten, lässt sich nach DE 39 10 543 allerdings ein lagerfähiges System erreichen, das erst unter Einwirkung von Hautfeuchtigkeit in einen übersättigten Zustand gerät und damit erst bei Anwendung den erwünschten erhöhten Wirkstofffluss auslöst.

Leider ist die Problemlösung nach dem vorstehenden Stand der Technik immer noch mit Nachteilen versehen. So erfordert die in DE 39 10 543 vorgestellte Lösung die Einbettung des Wirkstoffes in gelöster (fest-gelöster) Form. Damit ist für empfindliche Wirkstoffe die Gefahr der vorzeitigen Inaktivierung durch chemischen Abbau verbunden, denn Wirkstoffe sind in Lösung weniger stabil als in fester kristalliner Form (US 5,716,636). Des Weiteren ist das Ausmaß und der zeitliche Verlauf der Übersättigung schwer einstellbar, da diese stark vom Quellungsgrad der Einschlüsse (Inseln) im Zusammenhang mit der obligatorischen, den Feuchtezutritt begrenzenden Schicht abhängig sind. Ein weiterer Nachteil dieses Standes der Technik ist, erneut aufgrund der Erfordernis, eine gelöste Innenphase des Wirkstoffes bereitzustellen, die Notwendigkeit eines vergleichsweise hohen Hilfsstoffeinsatzes für das Basismaterial der Inseln, da sonst schwer lösliche Arzneistoffe nicht in feste Lösung gebracht werden können. Dies macht flexible und dünne Pflaster, die von Konsumenten bzw. Patienten bevorzugt werden, konstruktiv schwierig bis unmöglich.

WO 00/47191 offenbart ein Wirkstoffhaltiges Laminat für Transdermale Systeme, wobei eine Wirkstoffeinbettung in eine Polymere Klebematrix eingebracht ist. Diese Wirkstoffeinbettung besteht aus einem hydrophilen unvernetzten Polymer und einem in dieses Polymer eingebetteten wirkstoff.

Aufgabe der nachstehend im Einzelnen beschriebenen Erfindung ist daher die Bereitstellung von transdermalen therapeutischen Systemen mit erhöhtem Wirkstofffluss und gegenüber dem Stand der Technik verbesserter Stabilität, verbesserter Gleichmäßigkeit in der Bereitstellung des erhöhten Wirkstoffflusses und geringerem Hilfsstoffaufwand.

Diese Aufgabe wird erfindungsgemäß gelöst durch ein transdermales therapeutisches System, welches bevorzugt eine die Wasserdiffusion begrenzende, nach außen gewandte Rückschicht, und innerhalb einer zusammenhängenden, im wesentlichen wasserunlöslichen Außenphase (Basismaterial) befindliche, voneinander getrennte zahlreiche Einschlusskörper (Inseln), welche aus einem wasserlöslichen oder wasserquellbaren Material bestehen und den überwiegenden Teil des Wirkstoffgehaltes der Formulierung enthalten, der wiederum zum überwiegenden Teil in partikulärer, mikropartikulärer oder nanopartikulärer Zustandsform vorliegt, aufweist.

Als wesentliche Bestandteile des Basismaterials sind Polymere wie Kautschuk, kautschukähnliche synthetische Homo-, Co- oder Blockpolymere, Polyacrylsäure Ester und deren Copolymerisate, Polyurethane Copolymere des Ethylens, Polyisobutylen, Polybutylen und Polysiloxane beispielhaft zu nennen. Grundsätzlich kommen alle Polymere in Frage, die im Wesentlichen wasserunlöslich sind und im direkten und indirekten Kontakt mit der Haut keine nachteiligen Wirkungen auf den Menschen ausüben.

Da der klebende Verbund auch über eine zusätzlich angebrachte Klebschicht erfolgen kann, muss das Basismaterial nicht unbedingt primär haftklebend eingestellt sein, jedoch ist diese Eigenschaft für einen besonders dünnen und flexiblen, nicht auftragenden Systemaufbau, der auch ein einschichtiges System ermöglichen würde, bevorzugt. Weitere, dem Fachmann bekannte Stoffe mit funktionalem Einfluss auf das Basismaterial können eingesetzt werden, wie z.B. Weichmacher, Klebrigmacher, Resorptionsvermittler, Stabilisatoren oder Füllstoffe.

Als geeignete Hilfsstoffe für den Aufbau der wirkstoffhaltigen Einschlusskörper sind zunächst wasserlösliche oder wasserquellbare Polymere einsetzbar. Unter diesen sind beispielhaft zu nennen: Polyvinylalkohol und seine Copolymere, Polyvinylpyrrolidon und seine Copolymere, Polyethylenglycole, bevorzugt mit einem Molekulargewicht von über 1000 Dalton (die somit bei Raumtemperatur fest sind). Die vorstehenden Polymere können mit Vorteil für die kontrollierte Dispergierung der Einschlusskörper im Basismaterial bereits aus partikulären quervernetzten Strukturen bestehen. Weitere gut einsetzbare Polymere sind Alginate, Pullulan, Guar Gummi mit Gummi arabicum oder andere pflanzliche Gummen, Cellulose, insbesondere mikrokristalline Cellulose und ihre Derivate wie z.B. Methylcellulose, Hydroxyethylcellulose, Hydroxymethylpropylcellulose etc., aber auch andere Kohlenhydrate wie z.B. Stärke, besonders bevorzugt in derivatisierter oder modifizierter Form. Durchaus kommen aber auch peptidische Polymere wie Kollagen und Gelatine in Betracht. Wasserlösliche und wasserquellbare Polymere haben den Vorteil, dass sie bei Wasseraufnahme nicht plötzlich, sondern erst allmählich duktiler und diffusibler werden und somit den oder die eingeschlossenen Wirkstoff(e) gleichmäßiger abgeben. Dies ist insbesondere in Anwendungsfällen sinnvoll, bei denen die dispergierten Wirkstoffpartikel erst schrittweise in den Wirkstoffabgabeprozess einbezogen werden sollen.

Sollte ein rascherer Übergang bevorzugt werden, sind als alleinige oder zugemischte Hilfsstoffe für den Aufbau der wirkstoffhaltigen Einschlusskörper kleiner molekulare, wasserlösliche Stoffe vorteilhaft heranzuziehen. In erster Linie kommen hier wegen ihrer Eigenschaft, glasartig erstarrende, diffusionsfeste Partikel zu bilden, Zucker und ihre Derivate in Frage, vorrangig Saccharose, Glucose, Lactose, Fructose, aber auch Zuckeralkohole, wie Sorbit oder Mannit. Grundsätzlich eignen sich auch alle pharmazeutisch kompatiblen wasserlöslichen Stoffe, welche die Eigenschaft haben, sich unter einer Wasserdampfspannung von etwa 98 Prozent relativer Feuchte (wie sie von der Haut bereitgestellt wird) zu verflüssigen, wie z.B. Kochsalz, Harnstoff, Äpfelsäure, Zitronensäure.

Zusätze zur Erzielung von dem Fachmann bekannten weiteren Funktionalitäten wie z.B. Stabilisatoren (insbesondere Antioxidantien), Füllstoffen, aber auch mizellar agierenden Modifikatoren (Lecithine) können nach jeweiligem Erfordernis vorgesehen werden.

Da für den erfinderischen Zweck wesentlich ist, dass die Einschlusskörper sich als isolierte interne Phase im Basismaterial / Außenphase bilden, können als Phasenbildner korpuskulare hydrophile Partikel zugesetzt werden, wie feinverteiltes Kieselgel, nanodisperses Siliciumdioxid, Calciumsulfat, aber auch Polymere wie Cellulose-Derivate und andere z.B. weiter oben schon als mögliche Polymere für Einschlusskörper bereits genannte Substanzen.
Neben dem erfindungswesentlichen Komplex aus Basismaterial und Einschlusskörper, der im einfachsten Fall zusammen mit einer Rückschicht bereits ein komplettes TTS-System bilden kann, sind weitere Systembestandteile, die der Fachwelt bekannt sind, mit dem erfinderischen Prinzip sinnvoll kombinierbar.

Somit kann das erfindungsgemäße TTS, bevorzugt in Form eines trandermalen Pflasters, grundsätzlich aufgebaut sein wie aus dem Stand der Technik bekannte Systeme. Der erfindungswesentliche Unterschied besteht in dem erfindungsgemäß verbesserten Wirkstoffreservoir (Wirkstoffmatrix) aus im wesentlichen wasserunlöslichem Basismaterial, welches wasserlösliche oder wasserquellbare Einschlusskörper aufweist, die die Wirkstoffpartikel (partikuläre Phase) enthalten.

Unter den oben genannten weiteren Systembestandteilen sind z.B. polymerhaltige Schichten oder auch Membranen zu nennen, welche eine den Wirkstoffzufluss zur Haut kontrollierende Eigenschaft haben können oder auch die allzu rasche Aufnahme von Feuchtigkeit aus der Haut moderieren können.

Als Materialien für solche Membranen sind Polyethylen, Polyamid, Ethylenvinylacetat-Copolymere, aber auch mit niedermolekularen Stoffen gefüllte poröse Schichten üblich und dem Fachmann bekannt. Ohne oder mit Verwendung einer Membran können auch zusätzliche Klebschichten zur besseren hautseitigen Fixierung angebracht werden, deren wesentliche Hilfsstoffe bei der Erläuterung der Basismaterialien weiter oben bereits genannt sind. Hier sind besonders bevorzugt hoch diffusible lipophile Polymere zu nennen wie z.B. Polysiloxane und Acrylatcopolymere. Das erfindungsgemäße Prinzip kann darüber hinaus mit weiteren Methoden der Resorptionsverstärkung kombiniert werden. So können Penetrationsverstärker zugesetzt werden, welche die Durchlässigkeit der Haut erhöhen und physikalische Prinzipien, wie lontophorese, Elektroporation oder auch Ultraschall sowie Mikronadeln eingesetzt werden.

Als geeignete Wirkstoffe für die Wirkstoffpartikel kommen bevorzugt Stoffe zum Einsatz, deren Schmelzpunkt oberhalb von 50 °C liegt. Hier sind beispielhaft Atropin, Chlorpromazin, Haloperidol, Ephedrin, Propranolol, Clonidin, Moxonidin, Fentanyl, Indomethacin, Ethinylestradiol, Desogestrel, Testosteron, Granisetron, Pramipexol, Tetrahydrocannabinol, Vinpocetin neben vielen anderen Stoffen zu nennen. Darüber hinaus sind aber auch bei Raumtemperatur flüssige Wirkstoffe geeignet, sofern sie in eine kolloidale Form zu überführen sind. In diese Gruppe fallen beispielsweise Nikotin, Nitroglycerin, Selegilin, Bupropion.

Diese Aufzählung ist nicht erschöpfend, da grundsätzlich alle pharmazeutisch für die transdermale Gabe geeigneten Wirkstoffe aus zahlreichen Indikationsgruppen einsetzbar sind. Besonders bevorzugt sind Wirkstoffe, deren Sättigungsfluss auf der Haut ohne die Anwendung von weiteren Prinzipien der Resorptionsverstärkung nicht ausreicht. Zur Ermittlung dieser, besonders geeigneten Wirkstoffe, wird der Fachmann Vorversuche mit Permeationsuntersuchungen an isolierten Hautstücken vornehmen, indem er die bei Sättigung in einem inerten Medium festzustellende, quadratzentimeterbezogene Abgaberate bestimmt. Als besonders bevorzugte Kandidaten können mithin solche Wirkstoffe selektiert werden, deren Sättigungsfluss (also die Abgaberate ohne Anwendung des erfinderischen Prinzips), berechnet auf eine kommerziell relevante Systemfläche von 30 cm², 50% oder weniger der therapeutisch erforderlichen Dosierung ausmacht.

Die Korngröße der Wirkstoffpartikel ist grundsätzlich frei, solange sie deutlich (bevorzugt weniger als 20%, besonders bevorzugt weniger als 10% und ganz besonders bevorzugt weniger als 5%) unter der Partikelgröße der Einschlusskörper (diese liegt typisch bei kleiner/gleich 50 µm, bevorzugt bei 10-50 µm) liegt. Der volle erfinderische Vorteil wird bei besonders geringen Partikelgrößen des Wirkstoffs dadurch erreicht, dass dann ein Anstieg der Sättigungslöslichkeit und damit der thermodynamischen Aktivität ausnutzbar ist.

Besondere Vorteile der vorliegenden Erfindung ergeben sich auch aus der Stabilisierung von sehr fein zerteilten Wirkstoffpartikeln. In diesem Zusammenhang ist zu erwähnen, dass das bekannte Phänomen der "Ostwald-Reifung" sonst eine physikalische Destabilisierung zur Folge hat:
Fein verteilte mikronisierte oder sogar nanoskalige Partikel neigen zu einer Partikelvergröberung mit der Folge verringerter Oberflächenenergie. Dieses Phänomen ist insbesondere dann zu beobachten, wenn die Wirkstoffpartikel durch ein diffusives Medium miteinander verbunden sind und dann große Partikel auf Kosten von kleineren, die sich dann auflösen, wachsen können.

Durch die erfindungsgemäße Bereitstellung eines durch Trocknung bzw. Wasser oder Lösemittelentzug schwer diffusibel gewordenen Hilfsstoff-Komplexes der Einschlusskörper bleiben die transdermalen therapeutischen Systeme während der Lagerung vor dem Effekt der Ostwald-Reifung / Rekristallisation weitgehend geschützt. Somit ist es möglich, auch Partikel, die deutlich unter 1-10 µm, bevorzugt sogar unter 50 nm Durchmesser liegen, lagerstabil unterzubringen. Sobald dieses System auf der Haut angewandt wird, wirkt sich die erhöhte, thermodynamisch bedingte Sättigungslöslichkeit der Partikel in Bezug auf die Erhöhung des Wirkstoffflusses positiv aus. Auch die Beobachtung der erhöhten Sättigungslöslichkeit (nicht nur Lösungsgeschwindigkeit) solch kleiner Partikel geht auf Ostwald zurück und wird durch das "Ostwald-Freundlich-Gesetz" formelmäßig beschrieben ("Physikalische Chemie", VEB deutscher Verlag für Grundstoffindustrie, Leipzig 1974, S. 384).

Methoden zur Erzeugung solch kleiner Korngrößen bei Wirkstoffkristallen sind dem Fachmann bekannt und nicht kritisch für die Funktionalität der Erfindung. Möglichkeiten der kontrollierten Präzipitation können als pharmazeutische Grundoperationen eingestuft werden und ergeben sich beispielsweise aus der Mischung einer gesättigten Wirkstofflösung in einem Lösemittel, dem stufenweise unter ständiger Vermischung ein schlechteres Lösemittel zugesetzt wird.

Die entstehenden Partikel können nahezu auf jeder Stufe, selbst schon auf der Kolloid-Stufe, idealerweise schon unter Zugabe der später als Einschlusskörper dienenden Stoff- oder Stoffgemische durch Entfernen des Lösungsmittels (Trocknung, Sprühtrocknung, Flächentrocknung) erzeugt werden. Andere Erzeugungsmöglichkeiten für nanoskalige oder mikroskalige Wirkstoffe ergeben sich aus Techniken der Perlmühlenvermahlung oder der Homogenisierung von Partikeln in wässriger oder nicht-wässriger Umgebung. Beispielhaft sei für eine kurze Zusammenstellung auf Bushrab und Müller, (New Drugs, Ausgabe 5, 2003) verwiesen, aber auch andere Verfahren, wie die der Erzeugung durch überkritisches Kohlendioxid (Kümmel et al, GIT Labor-Fachzeitschrift 5/99, (1999) S. 511-514) können angewandt werden.

Die Rückschicht transdermaler Systeme für den erfindungsgemäßen Zweck kann z.B. in einer wasserdampfsperrend-okklusiv wirkenden Polyester (Polyethylenterephthalat-) Membran bestehen, die sowohl vor Wirkstoffverlust als auch vor Feuchtigkeitsverlust schützt. Durch entsprechende Dickenanpassung oder Wahl anderer Materialien (Polyethylen, Polyurethan, oder Laminate verschiedener thermoplastischer Rohstoffe) kann eine Moderation des Wasserdampfverlustes und damit eine genaue Einstellung des resultierenden Quellungs- oder Lösungszustandes der Einschlusskörper erfolgen.

Die Herstellung der erfindungsgemäßen Systeme selbst ist auf mannigfaltige Weise möglich. Besonders hervorgehoben und bevorzugt, aber letztlich beispielhaft sind folgende Möglichkeiten, die insbesondere die Herstellung des erfindungsgemäßen Wirkstoffreservoirs (Basismaterial mit wirkstoffhaltigen Einschlusskörpern) betreffen.

Ansonsten kann der Aufbau / Herstellung der erfindungsgemäßen TTS (Schichtaufbau, Materialien, Hilfs- und Zusatzstoffe) wie beschrieben nach den dem Fachmann aus dem Stand der Technik bekannten Methoden erfolgen (s. z.B. "Dermatological Formulation and Transdermal Systems", Kenneth A. Walters and Keith R. Brain in Dermatological and Transdermal Formulations, NY 2002, Marcel Dekker, Seite 319-399):
1. Dispergierung der mikronisierten oder nanoskaligen Wirkstoffpartikel in einer wässrigen Lösung des oder der Hilfsstoffe für den Aufbau der wirkstoffhaltigen Einschlusskörper, die eine Auflösung der Wirkstoffpartikel vermeidet und anschließende Trocknung. Die Trocknung kann z.B. durch Sprühtrocknung erfolgen, wobei gleich fein zerteilte Partikel erhalten werden oder aber durch Flächentrocknung mit anschließender Zerkleinerung der Partikel. Die so gewonnenen Einschlusskörper werden einer in organischer Lösung befindlichen oder sogar lösemittelfrei (Heißschmelzschnellverfahren) erzeugten Lösung oder Suspension des Basismaterials zugeführt, woraufhin nach anschließender Beschichtung auf die Rückschicht und Trocknung der Schicht ein nach Stanzung bereits funktionsfähiges Produkt erhalten wird. Das Wirkstoffreservoir (Basismaterial und Einschlusskörper) ist hier selbstklebend ausgebildet.
2. Die Formulierung der festen Einschlusskörper zusammen mit dem Wirkstoff kann auch bereits in Gegenwart des Basismaterials erreicht werden. Hierzu wird eine mit Wasser nicht mischbare organische Lösung des Basismaterials durch Rühren erzeugt, und in dieser eine Lösung oder Dispersion der Hilfsstoffe für den Aufbau der Einschlusskörper (einschließlich Wirkstoff) in einer wässrigen oder zumindest aus polarerem Lösemittel bestehenden Mischung erzeugt, die in der Lösung des Basismaterials dispergiert wird. Die Flüssig / Flüssig-Dispersion wird anschließend ebenfalls auf die Rückschichtfolie in gleichmäßiger Schichtdicke beschichtet. Durch den anschließenden Trockenprozess verfestigen sich die Einschlusskörper unter Lösemittelverlust. Durch geeignete Steuerung des Trocknungsverfahrens wird erreicht, dass in der letzten Stufe der Lösemittelentfernung sich in den Einschlusskörpern nano-oder mikroskalige Fällungsformen des Wirkstoffes bilden, deren weiteres Partikelwachstum durch den Abschluss des Trockenprozesses unterbunden wird.

Eine Variante dieses zweiten Verfahrens ist die Zugabe bereits nanoskaliger Wirkstoffe zum gesamten Lösemittelgemisch. Eine bevorzugte Anreicherung der Wirkstoffkristalle in den vorformulierten, noch lösemittelhaltigen Einschlusskörpern erfolgt regelmäßig durch die aufgrund der Polarität verbesserte Benetzbarkeit in der Innenphase.

Die genaue Wahl der Dimensionierungen von Schichtdicken und Polaritäten der einzelnen Systemkomponenten muss natürlich für jeden einzelnen Anwendungsfall gesondert festgelegt werden. Für das Zustandekommen des erfindungsgemäßen Vorteils sind zwei Steuerungsverfahren für das Ausmaß des erfindungsgemäß erhöhten Wirkstoffflusses festzuhalten:
1. Die Wahl der Korngröße des partikulären Wirkstoffgehaltes der Einschlusskörper, die nach der bereits erwähnten "Ostwald-Freundlich-Gleichung" eine entsprechende höhere Sättigungslöslichkeit zur Folge hat und
2. Der zusätzliche Effekt, der sich aus der durch Wärme erfolgenden Trocknung der Schichten des transdermalen Systems ergibt und eine wärmebedingte Übersättigung zur Folge haben kann.

### Beispiel 1

### Herstellung von Mikropartikeln:

2,5 g Gelatine werden in 100 ml 50 °C warmem Wasser gelöst. Danach werden 0,5 g Wirkstoffkristalle (Testosteron, mikronisiert) in der Gelatine Lösung suspendiert (4 Flügel Rührer, 500 - 1000 U/min). Anschließend wird die Suspension in ein Becherglas geschüttet, welches eine wässrige Lösung von Gummi Arabicum (2,5 %, w/v) enthält. 400 ml Wasser werden hinzugeführt, der pH-Wert wird mit Salzsäure (1 N) auf 3,0 - 4,3 gesenkt und die Mischung wird auf 4 °C abgekühlt. Nachdem Absetzen der Mikrokapseln für zwei Stunden, wird der Überstand dekantiert und die Partikel werden durch Zugabe von 2 x 150 ml Ethanol zum Sediment gehärtet. Zum Schluss werden die Mikrokapseln abfiltriert und über Nacht bis zur Massenkonstanz getrocknet.

Die hergestellten Partikel werden in 10,0 g Silikonkleber (z. B. Bio PSA 4201) suspendiert und zu einer Masse homogen gerührt. Diese Masse wird dann mit einer Handrakel auf eine flourpolymerisierte 100 µm PET- Folie in einer Schichtdicke von 50 bis 100 µm gestrichen und bei 30°C getrocknet und mit einer transparenten 15 µm PET - Folie kaschiert.

### Beispiel 2

0,5 g Testosteron werden in 10,0 g einer Lösung von Ethylcellulose in Ethanol (27,3%ig) gelöst. Die Lösung wird dann in 14,0 g Silikonkleber (z. B. Bio PSA 4201) suspendiert und zu einer Masse homogen gerührt. Diese Masse wird dann mit einer Handrakel auf eine flourpolymerisierte 100 µm PET- Folie in einer Schichtdicke von 50 bis 100 µm gestrichen und bei 80°C getrocknet und mit einer transparenten 15 µm PET - Folie kaschiert.

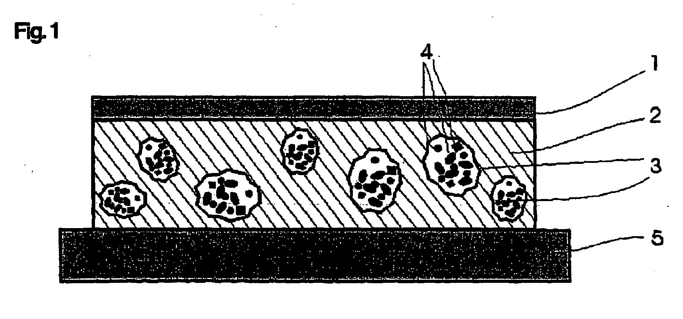

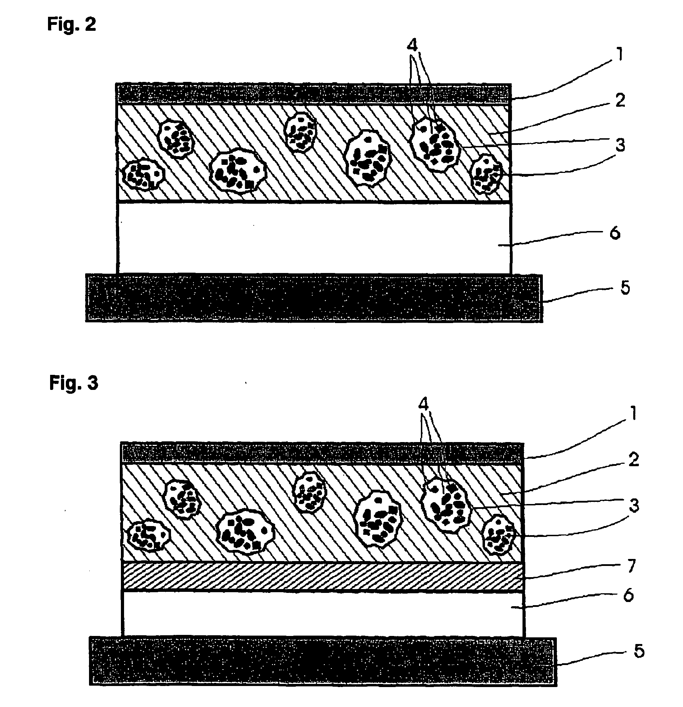

Die vorliegende Erfindung wird weiterhin beispielhaft durch die Figuren 1 bis 3 näher erläutert:
Fig. 1 zeigt ein erfindungsgemäßes TTS (transdermales Pflaster), das nur aus 2 Schichten besteht. Es bedeuten: (1) nach außen gewandte Rückschicht, (2) Basismaterial der aktiven - den Wirkstoff enthaltenden - Schicht, (3) Einschlusskörper, die die Wirkstoffpartikel (4) enthalten und (5) eine ablösbare Schutzfolie, die das TTS vor dem Gebrauch schützt. Nach dem Abziehen der Schutzfolie (5) wird das TTS mit dieser selbstklebenden Seite auf die Haut appliziert.
Fig. 2 zeigt ein erfindungsgemäßes TTS, welches eine zusätzliche Klebeschicht (6) aufweist.
Fig. 3 zeigt ein erfindungsgemäßes TTS, welches zusätzlich über eine Kontrollmembran (7) verfügt.

## Patentansprüche

1. Transdermales therapeutisches System (TTS) mit einer wirkstoffhaltigen Matrix enthaltend ein im wesentlichen wasserunlösliches Basismaterial, welches wasserlösliche oder wasserquellbare Einschlusskörper aufweist, die Wirkstoffpartikel als Mikro- oder Nanopartikel enthalten.

2. Transdermalen therapeutisches System nach Anspruch 1, **dadurch gekennzeichnet, dass** es einen Schichtaufbau hat.

3. Transdermales therapeutisches System nach Anspruch 2, **dadurch gekennzeichnet, dass** es zwei Schichten umfasst, eine nach außen gewandte Rückschicht und eine den Wirkstoff enthaltende Schicht.

4. Transdermales therapeutisches System nach Anspruch 2 oder 3, **dadurch gekennzeichnet, dass** es eine Klebeschicht aufweist.

5. Transdermalen therapeutisches System nach Anspruch 2, 3 oder 4, **dadurch gekennzeichnet, dass** es eine Kontrollmembran aufweist.

6. Transdermales therapeutisches System nach einem oder mehreren der Ansprüche 1 bis 5 **dadurch gekennzeichnet, dass** die Einschlusskörper eine Partikelgröße von kleiner als 50 mm aufweisen.

7. Transdermales therapeutisches System nach einem oder mehreren der Ansprüche 1 bis 6 **dadurch gekennzeichnet, dass** die Größe der Wirkstoffpartikel weniger als 10 % von der Partikelgröße der Einschlusskörper beträgt, bevorzugt liegt die Größe unter 50 nm.

8. Transdermales therapeutisches System nach einem oder mehreren der Ansprüche 1 bis 7 **dadurch gekennzeichnet, dass** der Wirkstoff einen Schmelzpunkt oberhalb von 50°C hat.

9. Transdermales therapeutisches System nach einem oder mehreren der Ansprüche 1 bis 8 **dadurch gekennzeichnet, dass** das Basismaterial der Wirkstoffmatrix Kautschuk, kautschukähnliche synthetische Homo-, Co- oder Blockpolymere. Polyacrylsäureester oder deren Copolymerisate, Polyurethane, Polyisobutylen, Polybutylen oder Polysiloxane enthält.

10. Transdermales therapeutisches System nach einen oder mehreren der Ansprüche 1 bis 9 **dadurch gekennzeichnet, dass** die Einschlusskörper Polyvinylalkohol oder dessen Copolymere, Polyvinylpyrrollidon oder dessen Copolymere, Polyethylenglykol, Alginat, Pullulan, Guar Gummi mit Gummi arabicum, Cellulose oder deren Derivate oder Stärke oder Stärkederivate enthalten.

11. Transdermales therapeutisches System nach einem oder mehreren der Ansprüche 1 bis 10, **dadurch gekennzeichnet, dass** der Wirkstoff Atropin, Chlorpromazin, Haloperidol, Ephedrin, Propranolol, Clonidin, Moxonidin, Fentanyl, Indomethacin, Ethinylestradiol, Desogestrel, Testosteron, Gramisetron, Pramipexol, Tetrahydrocannabinol oder Vinpocetin ist.

12. Transdermalen therapeutisches System nach einem oder mehreren der Ansprüche 1 bis 11, **dadurch gekennzeichnet, dass** die wirkstoffhaltige Matrix weitere Hilfs- oder Zusatzstoffe enthält.

13. Verfahren zur Herstellung der wirkstoffhaltigen Matrix eines transdermalen therapeutischen Systems nach einem oder mehreren der Ansprüche 1 bis 12, umfassend die Schritte:
a) Dispergierung der mikronisierten oder nanoskaligen Wirkstoffpartikel in einer Lösung der Hilfsstoffe für den Aufbau der Einschlusskörper,
b) Trocknen der Dispersion und zerkleinern der erhaltenen Partikel und
c) Einbringung der nach Schritt b) erhaltenen wirkstoffhaltigen Einschlusskörper in eine Lösung oder suspension des Basismaterials.

14. Verfahren nach Anspruch 13, wobei die Einbringung der Einschlusskörper in das Basismaterial gemäß Schritt c) lösernittelfrei geschieht.

15. Verfahren nach Anspruch 14, wobei die Einbringung der Einschlusskörper in das Basismaterial gemäß Schritt c) nach dem Heißschmelzschnellverfahren geschieht.

16. Verfahren zur Herstellung der wirkstoffhalfigen Matrix eines transdermalen therapeutischen Systems nach einem oder mehreren der Ansprüche 1 bis 12 umfassend die Schritte:
a) Herstellen einer Flüssig / Flüssig-Dispersion bestehend aus einer mit Wasser nicht mischbaren Lösung des Basismaterials und aus einer Lösung oder Dispersion des Wirkstoffs und der Hilfsstoffe für den Aufbau der Einschlusskörper in Wasser, einem polaren Lösungsmittel oder einer Mischung aus Wasser und polarem Lösungsmittel und
b) Trocknung der nach Schritt a) erhaltenen Dispersion.

17. Verfahren nach einem der Ansprüche 13 bis 16, **dadurch gekennzeichnet, dass** die Hilfsstoffe einen Phasenbildner umfassen.

18. Verwendung eines transdermalen therapeutischen Systems nach einem oder mehreren der Ansprüche 1 bis 12 zur Applikation von medizinischen Wirkstoffen über die Haut eines Patienten.

## Claims

1. A transdermal therapeutic system (TTS) with an active ingredient-containing matrix comprising an essentially water-insoluble base material which has water-soluble or water-swellable inclusion bodies which comprise active ingredient particles as micro- or nanoparticles.

2. The transdermal therapeutic system as claimed in claim 1, which has a layered structure.

3. The transdermal therapeutic system as claimed in claim 2, which includes two layers, a backing layer facing outward, and a layer comprising the active ingredient.

4. The transdermal therapeutic system as claimed in claim 2 or 3, which has an adhesive layer.

5. The transdermal therapeutic system as claimed in claim 2, 3 or 4, which has a control membrane.

6. The transdermal therapeutic system as claimed in one or more of claims 1 to 5, wherein the inclusion bodies have a particle size of less than 50 mm.

7. The transdermal therapeutic system as claimed in one or more of claims 1 to 6, wherein the size of the active ingredient particles is less than 10 % of the particle size of the inclusion bodies, the size is preferably below 50 nm.

8. The transdermal therapeutic system as claimed in one or more of claims 1 to 7, wherein the active ingredient has a melting point above 50 °C.

9. The transdermal therapeutic system as claimed in one or more of claims 1 to 8, wherein the base material of the active ingredient matrix comprises rubber, rubber-like synthetic homopolymers, copolymers or block polymers, polyacrylic acid esters or copolymers thereof, polyurethanes, polyisobutylene, polybutylene or polysiloxanes.

10. The transdermal therapeutic system as claimed in one or more of claims 1 to 9, wherein the inclusion bodies comprise polyvinyl alcohol or its copolymers, polyvinylpyrrolidone or its copolymers, polyethylene glycol, alginate, pullulan, guar gum with gum arabic, cellulose or derivatives thereof or starch or starch derivatives.

11. The transdermal therapeutic system as claimed in one or more of claims 1 to 10, wherein the active ingredient is atropine, chlorpromazine, haloperidol, ephedrine, propranolol, clonidine, moxonidine, fentanyl, indomethacin, ethinylestradiol, desogestrel, testosterone, granisetron, pramipexole, tetrahydrocannabinol or vinpocetine.

12. The transdermal therapeutic system as claimed in one or more of claims 1 to 11, wherein the active ingredient matrix comprises further excipients or additives.

13. A process for producing the active ingredient-containing matrix of a transdermal therapeutic system as claimed in one or more of claims 1 to 12, comprising the steps:
a) dispersing the micronized or nanoscale active ingredient particles in a solution of the excipients for building up the inclusion bodies,
b) drying the dispersion and comminuting the resulting particles and
c) introducing the active ingredient-containing inclusion bodies obtained in step b) into a solution or suspension of the base material.

14. The process as claimed in claim 13, where the introduction of the inclusion bodies into the base material in step c) takes place without solvent.

15. The process as claimed in claim 14, where the introduction of the inclusion bodies into the base material in step c) takes place by the hot-melt rapid process.

16. A process for producing the active ingredient-containing matrix of a transdermal therapeutic system as claimed in one or more of claims 1 to 12 comprising the steps:
a) producing a liquid/liquid dispersion consisting of a water-immiscible solution of the base material and of a solution or dispersion of the active ingredient and of the excipients for building up the inclusion bodies in water, a polar solvent or a mixture of water and polar solvent and
b) drying the dispersion obtained in step a).

17. The process as claimed in any of claims 13 to 16, wherein the excipients include a phase former.

18. The use of a transdermal therapeutic system as claimed in one or more of claims 1 to 12 for administering medicinal active ingredients via the skin of a patient.

## Revendications

1. Système thérapeutique percutané (TTS) avec une matrice, contenant un principe actif, contenant un matériau de base essentiellement insoluble dans l'eau, qui présente des corps d'inclusion solubles dans l'eau ou gonflant dans l'eau, qui contiennent des particules de principe actif sous forme de microparticules ou de nanoparticules.

2. Système thérapeutique percutané selon la revendication 1, **caractérisé en ce qu'**il possède une structure en couches.

3. Système thérapeutique percutané selon la revendication 2, **caractérisé en ce qu'**il comprend deux couches, une couche arrière tournée vers l'extérieur et une couche contenant le principe actif.

4. Système thérapeutique percutané selon la revendication 2 ou 3, **caractérisé en ce qu'**il présente une couche de colle.

5. Système thérapeutique percutané selon la revendication 2, 3 ou 4, **caractérisé en ce qu'**il présente une membrane de contrôle.

6. Système thérapeutique percutané selon l'une quelconque des revendications 1 à 5, **caractérisé en ce que** les corps d'inclusion présentent une taille de particules inférieure à 50 nm.

7. Système thérapeutique percutané selon l'une quelconque des revendications 1 à 6, **caractérisé en ce que** la taille des particules de principe actif sont inférieures à 10 % de la taille de particules des corps d'inclusion, et la taille est de manière préférée inférieure à 50 nm.

8. Système thérapeutique percutané selon l'une quelconque des revendications 1 à 7, **caractérisé en ce que** le principe actif possède un point de fusion supérieur à 50°C.

9. Système thérapeutique percutané selon l'une quelconque des revendications 1 à 8, **caractérisé en ce que** le matériau de base de la matrice de principe actif contient du caoutchouc, des homopolymères, copolymères ou polymères séquences synthétiques semblables au caoutchouc, des esters d'acide polyacrylique ou leurs produits copolymérisés, des polyuréthannes, du polyisobutylène, du polybutylène ou des polysiloxanes.

10. Système thérapeutique percutané selon l'une quelconque des revendications 1 à 9, **caractérisé en ce que** les corps d'inclusion contiennent de l'alcool polyvinylique ou ses copolymères, du polyvinylpyrrolidone ou ses copolymères, du polyéthylèneglycol, de l'alginate, du pullulane, de la gomme de guar avec de la gomme arabique, du cellulose ou ses dérivés ou des amidons ou des dérivés d'amidon.

11. Système thérapeutique percutané selon l'une quelconque des revendications 1 à 10, **caractérisé en ce que** le principe actif est de l'atropine, de la chlorpromazine, de l'halopéridol, de l'éphédrine, du propranolol, de la clonidine, de la moxonidine, du fentanyl, de l'indométhacine, de l'éthinyloestradiol, du désogestrel, de la testostérone, du granisétron, du pramipexole, du tétrahydrocannabinol ou de la vinpocétine.

12. Système thérapeutique percutané selon l'une quelconque des revendications 1 à 11, **caractérisé en ce que** la matrice contenant du principe actif contient d'autres adjuvants ou additifs.

13. Procédé de fabrication de la matrice contenant un principe actif d'un système thérapeutique percutané selon l'une quelconque des revendications 1 à 12, comprenant les étapes consistant à :
a) disperser les particules de principe actif micronisées ou à l'échelle nanométrique dans une solution des adjuvants pour la constitution des corps d'inclusion,
b) sécher la dispersion et microniser les particules obtenues et
c) mettre en place les corps d'inclusion contenant un principe actif et obtenus à l'étape b) dans une solution ou une suspension du matériau de base.

14. Procédé selon la revendication 13, dans lequel la mise en place des corps d'inclusion dans le matériau de base a lieu selon l'étape c) en l'absence de solvant.

15. Procédé selon la revendication 14, dans lequel la mise en place des corps d'inclusion dans le matériau de base a lieu selon l'étape c) selon le procédé de thermofusion rapide.

16. Procédé de fabrication de la matrice contenant un principe actif d'un système thérapeutique percutané selon l'une quelconque des revendications 1 à 12 comprenant les étapes consistant à :
a) préparer une dispersion liquide/liquide constituée d'une solution, non miscible avec l'eau, du matériau de base et d'une solution ou d'une dispersion du principe actif et des adjuvants pour la constitution des corps d'inclusion dans de l'eau, un solvant polaire ou un mélange d'eau et de solvant polaire, et
b) sécher la dispersion obtenue après l'étape a).

17. Procédé selon l'une quelconque des revendications 13 à 16, **caractérisé en ce que** les adjuvants comprennent un générateur de phase.

18. Utilisation d'un système thérapeutique percutané selon l'une quelconque des revendications 1 à 12 pour l'application de principes actifs médicaux sur la peau d'un patient.
